# EUROPÄISCHE PATENTSCHRIFT

(11) **EP 1 212 992 B1**
(45) Veröffentlichungstag und Bekanntmachung des Hinweises auf die Patenterteilung: **15.08.2007**
(21) Anmeldenummer: 01127175.6
(22) Anmeldetag: 16.11.2001
(51) Int. Cl.: A61F 2/44, A61F 2/30

(54) **Implantat**
Implant
Implant

(30) Priorität: 17.11.2000 DE 10056977
(43) Veröffentlichungstag der Anmeldung: 12.06.2002
(73) Patentinhaber: Aesculap AG & Co. KG, 78532 Tuttlingen (DE)
(72) Erfinder: Kossmann M. D., Thomas. Prof. Dir., Monash Univ., MELBOURNE, VICTORIA 3181 (AU); Blömer, Wilhelm, 88690 Unteruhldingen-Mühlhofen (DE); Schultz, Robert, 78532 Tuttlingen (DE)
(74) Vertreter: Hoeger, Stellrecht & Partner Patentanwälte

(56) Entgegenhaltungen:
- EP-A- 0 346 269
- WO-A-00/35386
- DE-A- 2 203 242
- US-A- 4 932 975
- US-A- 5 702 450
- US-A- 6 132 465

## Beschreibung

Die Erfindung betrifft ein Implantat zum Einsetzen in einen Zwischenraum zwischen zwei Wirbelkörpern der Wirbelsäule mit zwei an den zu beiden Seiten des Zwischenraumes angeordneten Wirbelkörpern anlegbaren Stützelementen und mit einem zwischen diesen angeordneten, sich in Längsrichtung des Implantates erstreckenden rohrförmigen Hohlraum, der durch eine balgförmige, in Längsrichtung des Implantates verlängerbare und in Umfangsrichtung des Hohlraumes im wesentlichen nicht dehnbare Außenwand begrenzt wird.

Defekte in der Wirbelsäule werden häufig dadurch überbrückt, daß anstelle der aus der Wirbelsäule entfernten Körperteile Implantate eingesetzt werden. Es kann sich dabei um sogenannte Zwischenwirbelkörperimplantate handeln, die eine einzelne entfernte Bandscheibe ersetzen, es kann sich um Wirbelkörperersatzimplantate handeln, die entfernte Wirbelkörper insgesamt ersetzen, und es gibt Implantate, die sowohl entfernte Wirbelkörper als auch entfernte Bandscheiben ersetzen.

Ein Beispiel eines Implantates, welches einen Wirbelkörper und zwei daran anschließende Bandscheiben ersetzt, ist in der US 4,932,975 beschrieben. Der durch die Entfernung eines Wirbelkörpers und der beiden anliegenden Bandscheiben entstandene Zwischenraum wird dabei durch ein Implantat überbrückt, welches zwei an den gegenüberliegenden Endes des Implantates angeordnete Stützelemente aufweist und einen dazwischenliegenden Hohlraum, der durch einen metallischen Balg gebildet wird, wie er beispielsweise in Druckmeßdosen Verwendung findet. Die dabei verwendete Konstruktion ist relativ aufwendig, da eine größere Anzahl von Teilen miteinander kombiniert werden müssen, um die komplexen Funktionen dieses Implantates zu übernehmen.

Es ist Aufgabe der Erfindung, ein gattungsgemäßes Implantat so auszugestalten, daß es in sehr einfacher Weise herstellbar ist.

Diese Erfindung wird bei einem Implantat der eingangs beschriebenen Art erfindungsgemäß dadurch gelöst, daß die Außenwand von einem Schlauchstück aus einem dicht gewebten oder gewirkten textilen Material gebildet wird.

Es hat sich überraschenderweise herausgestellt, daß die Begrenzung des Hohlraumes zwischen den Stützelementen in sehr einfacher Weise durch ein solches textiles Schlauchstück vorgenommen werden kann. Derartige Schlauchstücke sind an sich bekannt und werden als Gefäßprothesen und als Einsätze in Blutgefäße (sogenannte Stents) verwendet, es handelt sich dabei um balgartig geformte Schlauchstücke, bei denen die verwendeten Fäden derartig dicht gewebt oder gewirkt sind, daß diese Schlauchstücke so dicht werden, daß in ihnen aufgenommene Flüssigkeiten in ihrem Inneren zurückgehalten werden können. Es hat sich nun überraschenderweise herausgestellt, daß diese an sich bekannten Schlauchstücke geeignet sind, um in Verbindung mit zwei an Wirbelkörper anlegbaren Stützelementen ein Implantat auszubilden, welches zur Überbrückung von Defekten der Wirbelsäule geeignet ist.

Das Schlauchstück kann bei einer bevorzugten Ausführungsform der Erfindung aus Fäden aufgebaut sein, die aus Polyethylenterephtalat bestehen.

Bei einer bevorzugten Ausführungsform ist vorgesehen, daß das Schlauchstück an seinen Enden flüssigkeitsdicht mit den Stützelementen verbunden ist. Beispielsweise kann der Endbereich des Schlauchstückes in eine Umfangsnut der Stützelemente eingelegt sein und in dieser Position durch eine äußere Bandage fixiert werden.

Günstig ist es, wenn die Stützelemente plattenförmig ausgebildet sind und sich im wesentlichen quer zur Längsrichtung des Implantates erstrecken, der Hohlraum wird bei einer solchen Konstruktion an der Außenseite durch das textile Schlauchstück und an den beiden Stirnseiten durch die beiden Stützelemente begrenzt.

Es ist günstig, wenn die Stützelemente an ihrer jeweiligen Außenfläche, die einem Wirbelkörper benachbart ist, Vorsprünge tragen, die bei Anlage an dem Wirbelkörper in dessen Knochenmaterial eindringen und dadurch eine innige Verbindung zwischen Stützelement und Wirbelkörper ermöglichen. Diese Vorsprünge können makroskopische Vorsprünge sein, beispielsweise nebeneinanderliegende Dorne oder Rippen, es ist aber auch möglich, daß es sich um mikroskopische Vorsprünge handelt, die beispielsweise durch ein Aufrauhen der Außenfläche oder durch die Beschichtung der Außenfläche mit einer rauhen Oberfläche erzielt werden können.

Bei einer bevorzugten Ausführungsform ist vorgesehen, daß die Stützelemente Durchbrechungen aufweisen, durch die der Hohlraum mit der Außenfläche der Stützelemente verbunden ist. Durch diese Durchbrechungen kann Füllmaterial aus dem Hohlraum in den Anlagebereich der Stützelemente an dem Wirbelkörper eintreten und dort zu einer optimalen Verbindung beitragen.

Der Hohlraum kann einen Zufluß für ein fließfähiges Füllmedium aufweisen.

Bei einer ersten bevorzugten Ausführungsform ist vorgesehen, daß der Hohlraum mit einer dauerhaft fließfähigen oder einer elastischen Substanz gefüllt ist. Ein in dieser Weise ausgebildetes Implantat wird vorzugsweise als Bandscheibenersatz eingesetzt, da bei diesem Implantat die beiden einander gegenüberliegenden Stützelemente in der Art einer Bandscheibe gegeneinander verschwenkt werden können und auch eine Stoßabsorptionsfunktion ausüben können. Die freie Verschwenkbarkeit der benachbarten Stützelemente wird dabei durch die spezielle Ausgestaltung der Außenwand des Hohlraumes erleichtert, das textile Schlauchstück läßt ohne weiteres ein Verschwenken, ein Verdrehen um die Längsachse des Implantates und sogar in geringem Umfange eine seitliche Versetzung der beiden Stützelemente zu, während in radialer Richtung durch die fehlende Dehnbarkeit des Schlauchstückes in Umfangsrichtung die Füllung des Hohlraumes dauerhaft begrenzt wird und nicht radial ausgeweitet wird.

Die dauerhaft fließfähige Substanz kann beispielsweise ein Hydrogel sein, als elastische Substanz kann Silikon eingefüllt sein.

Bei einer anderen Ausführungsform ist vorgesehen, daß der Hohlraum mit einer fließfähigen aushärtbaren Substanz gefüllt ist. Ein in dieser Weise ausgebildetes Implantat ist insbesondere als Wirbelersatzkörper geeignet. Bei diesem Implantat werden die Stützelemente nach dem Einsetzen in den zu überbrückenden Zwischenraum mit einem geeigneten Instrument aufgespreizt und gegen die an den Zwischenraum angrenzenden Wirbelkörper angelegt, sobald die erwünschte Erstrekkung des Implantates in dessen Längsrichtung erreicht ist, wird der Hohlraum mit der fließfähigen, aushärtbaren Substanz aufgefüllt, die dann nach der Aushärtung den Zwischenraum dauerhaft und axial unveränderlich überbrückt.

Die fließfähige, aushärtbare Substanz kann Knochenzement sein, beispielsweise ein Knochenzement auf der Basis eines Acrylharzes.

Die in dieser Weise beschriebenen, einen Hohlraum aufweisenden Implantate können je nach Füllsubstanz als Ersatz für eine Bandscheibe oder als Ersatz für einen Wirbelkörper eingesetzt werden, wobei die Erstreckung des Implantates in Längsrichtung des Implantates entsprechend den natürlichen Abmessungen einer Bandscheibe und eines Wirbelkörpers gewählt werden, das heißt ein Bandscheibenersatzimplantat wird in Längsrichtung eine wesentlich geringere Erstreckung aufweisen als ein Wirbelkörperersatzimplantat, beispielsweise wird die Längserstreckung eines Bandscheibenersatzimplantates in der Größenordnung von 10 bis 15 mm liegen, die Längserstreckung eines Wirbelkörperersatzimplantates in der Größenordnung von 25 bis 70 mm pro ersetztem Wirbelkörper.

Bei einer weiteren bevorzugten Ausführungsform eines derartigen Implantates ist vorgesehen, daß sich in Längsrichtung des Implantates zwischen den Stützelementen mindestens zwei Hohlräume befinden, die von einer schlauchförmigen Außenwand aus einem dicht gewebten oder gewirkten textilen Material begrenzt werden.

Derartige Implantate mit mehreren Hohlräumen können eingesetzt werden, um sowohl die Funktion einer Bandscheibe als auch die Funktion eines Wirbelkörpers zu übernehmen und somit sowohl Bandscheiben als auch Wirbelkörper zu ersetzen.

So ist beispielsweise möglich, daß bei einem Implantat ein Hohlraum mit einer dauerhaft fließfähigen oder einer elastischen Substanz gefüllt ist und der andere mit einer fließfähigen, aushärtbaren Substanz. Ein solches Implantat kann einen Wirbelkörper und eine angrenzende Bandscheibe ersetzen.

Besonders vorteilhaft ist ein symmetrischer Aufbau, bei dem ein Implantat drei in Längsrichtung aneinander anschließende Hohlräume aufweist. Der mittlere Hohlraum ist dabei mit einer fließfähigen aushärtbaren Substanz gefüllt und die beiden äußeren Hohlräume mit einer dauerhaft fließfähigen oder elastischen Substanz. Ein solches Implantat kann einen Wirbelkörper mit beiden daran anschließenden Bandscheiben ersetzen.

Entsprechend der anatomischen Erstreckung eines Wirbelkörpers und der angrenzenden Bandscheiben ist dabei vorgesehen, daß die Ausdehnung des mit einer aushärtbaren Substanz gefüllten Hohlraums in Längsrichtung des Implantates größer ist als die des mit einer dauerhaft fließfähigen oder elastischen Substanz gefüllten Hohlraumes.

Die nachfolgende Beschreibung bevorzugter Ausführungsformen der Erfindung dient im Zusammenhang mit der Zeichnung der näheren Erläuterung. Es zeigen:
- Figur 1:: eine schematische Seitenansicht eines als Wirbelersatzkörper dienenden Implantates;
- Figur 2:: eine Schnittansicht längs Linie 2-2 in Figur 1;
- Figur 3:: eine Ansicht ähnlich Figur 1 mit dem Implantat in Längsschnittdarstellung;
- Figur 4:: eine schematische Längsschnittdarstellung eines als Bandscheibenersatz dienenden Implantates und
- Figur 5:: eine schematische Längsschnittansicht eines Wirbelkörperersatzimplantates, welches gleichzeitig die Funktion von zwei benachbarten Bandscheiben übernimmt.

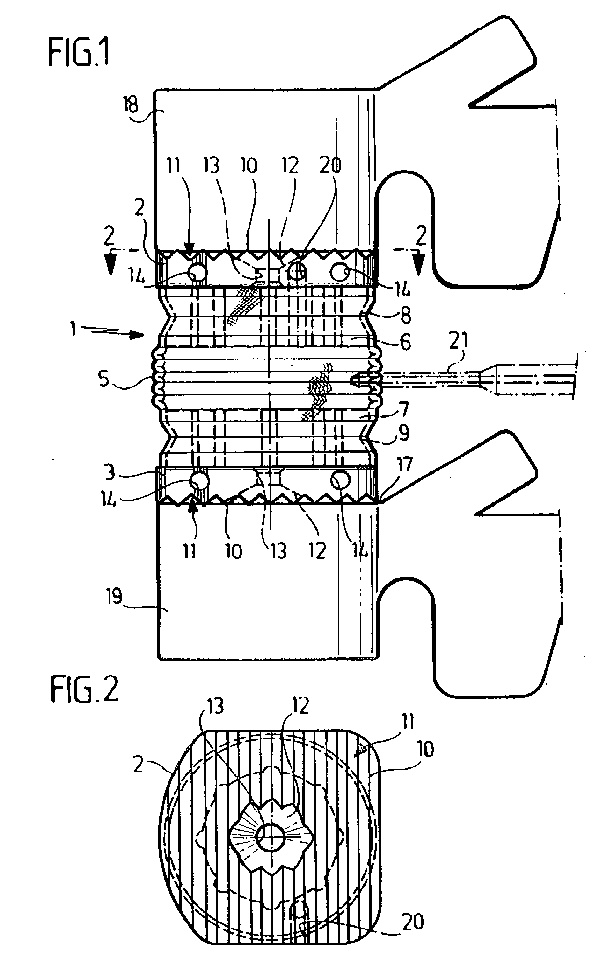

Das in den Figuren 1 bis 3 dargestellte Implantat 1 umfaßt zwei plattenförmige Stützelemente 2, 3, die an gegenüberliegenden Enden des Implantates 1 angeordnet sind und zwischen sich einen Hohlraum 4 einschließen, der durch eine flexible Außenwand 5 seitlich begrenzt wird. Die flexible Außenwand 5 besteht aus einem balgförmigen Schlauchstück aus einem textilen Material, beispielsweise aus Polyethylenterephtalat, bei dem die Fäden sehr dicht gewebt oder gewirkt sind, so daß diese Außenwand 5 im wesentlichen flüssigkeitsdicht ist. Die Außenwand 5 kann dabei einlagig oder mehrlagig ausgebildet sein, wesentlich ist, daß durch die Verwendung von Textilmaterial und die balgförmige Ausgestaltung die Außenwand 5 in Längsrichtung des Implantates, also in Richtung von einem Stützelement 2 zum anderen Stützelement 3, verlängerbar ist, in Umfangsrichtung jedoch im wesentlichen nicht oder gar nicht dehnbar.

Die beiden plattenförmigen Stützelemente 2 und 3 tragen auf den einander zugewandten Seiten in gleicher Weise kragenförmige Vorsprünge 6, 7, die jeweils eine Umfangsnut 8 beziehungsweise 9 aufweisen, in diese Umfangsnuten 8 und 9 sind die Endbereiche der schlauchförmigen Außenwand 5 eingelegt und dort mit Hilfe einer in der Zeichnung nicht dargestellten Bandage festgelegt, so daß der Hohlraum 4 stirnseitig durch die beiden Stützelemente 2 und 3 und mantelseitig durch die Außenwand 5 begrenzt wird.

Die beiden Stützelemente 2 und 3 sind gleich aufgebaut und in entgegengesetzter Orientierung in das Implantat 1 eingebaut. Sie weisen eine mit rippenförmigen Vorsprüngen 10 versehene Außenfläche 11 auf, in dieser Außenfläche 11 befindet sich eine zentrale Vertiefung 12, die über einen Verbindungskanal 13 mit dem Hohlraum 4 in Verbindung steht.

Die Stützelemente 2 und 3 bestehen bevorzugt aus Metall, beispielsweise aus Titan.

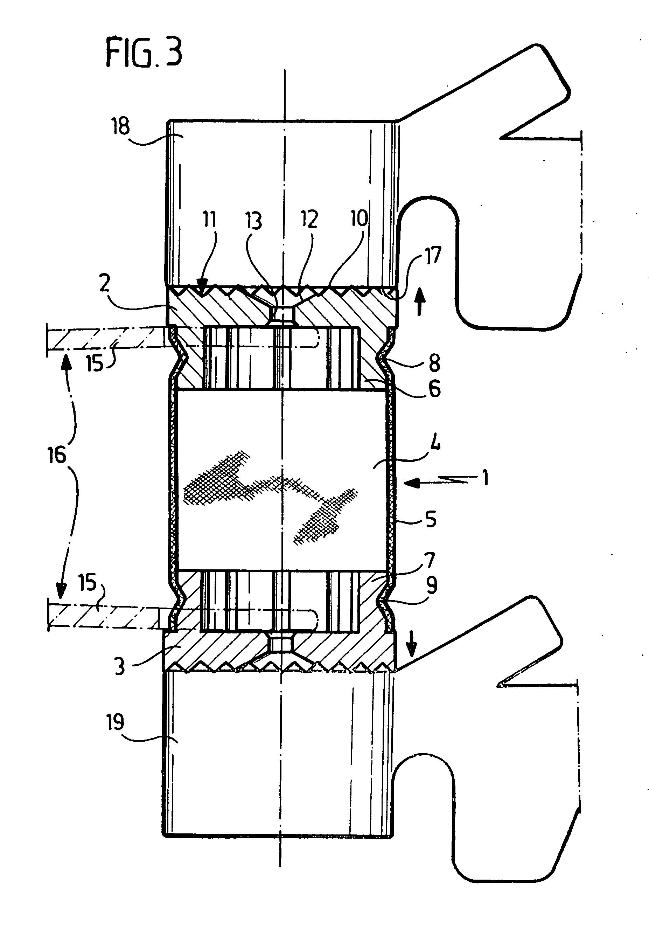

In die Stützelemente 2 und 3 sind zur Seite hin offene, quer zur Längsrichtung des Implantates 1 verlaufende Aufnahmeöffnungen 14 eingearbeitet, in die entsprechende Werkzeuge 15 eines Spreizinstrumentes 16 eingeführt werden können, dies ist in Figur 3 in strichpunktierten Linien angedeutet. Mit Hilfe dieses Spreizinstrumentes 16 ist es möglich, den Abstand der Stützelemente 2 und 3 voneinander zu verändern, dabei wird die balgförmig zusammengefaltete Außenwand 5 in Längsrichtung des Implantates 1 gestreckt.

Das beschriebene Implantat 1 dient als Wirbelkörperersatzimplantat. Zu diesem Zweck wird das Implantat 1 in den Zwischenraum 17 zwischen zwei Wirbelkörpern 18 beziehungsweise 19 eingeführt. Dieser Zwischenraum 17 ist dadurch entstanden, daß ein Wirbelkörper und die benachbarten Bandscheiben entfernt worden sind. Nach dem Einführen des Implantates in den Zwischenraum 17 werden die beiden Stützelemente 2 und 3 mit Hilfe eine Spreizinstrumentes 16 so gegen die Wirbelkörper 18 und 19 bewegt, daß sich die Stützelemente 2 und 3 mit ihren Außenflächen 11 an die beiden Wirbelkörper 18 und 19 anlegen. Das Implantat wird dabei so weit gestreckt, daß sich die beiden Wirbelkörper 18 und 19 etwa in der Position befinden, in der sie sich vor Entfernung des Wirbelkörpers und der Bandscheiben aus dem Zwischenraum 17 befanden, also in natürlicher Position.

In dieser Lage der Stützelemente 2 und 3 wird der Hohlraum 4 mit einer fließfähigen, aushärtbaren Substanz gefüllt, beispielsweise mit einem Knochenzement auf Acrylharzbasis. Das Einführen kann entweder erfolgen über einen Einführkanal 20 in einem Stützelement 2 oder durch eine Öffnung in der Außenwand 5. Diese Öffnung kann beispielsweise durch einen kleinen Schnitt erzeugt werden oder durch die Injektionsnadel 21 einer Einfüllvorrichtung. Das in den Hohlraum eingefüllte fließfähige Material füllt diesen Hohlraum vollständig aus, in radialer Richtung wird dieses Material durch die Außenwand auf den Querschnitt lokalisiert, der durch die in Umfangsrichtung nicht dehnbare Außenwand 5 definiert wird, in axialer Richtung wird der Abstand der beiden Stützelemente 2 und 3 durch das Spreizinstrument 16 aufrechterhalten, so daß durch die Füllung keine Verschiebung der Stützelemente 2 und 3 mehr eintritt. Die eingefüllte Substanz härtet nach vollständiger Ausfüllung des Hohlraumes 4 aus und bildet somit einen von der Außenwand 5 umgebenen im wesentlichen starren Verbindungsteil aus, welcher die beiden Stützelemente 2 und 3 in der einmal eingenommenen Position dauerhaft festlegt. Das Implantat aus den beiden Stützelementen 2 und 3 und dem mit dem ausgehärteten Material gefüllten Hohlraum 4 bildet somit ein weitgehend starres Zwischenstück aus, welches den gesamten Zwischenraum 17 ausfüllt und die Wirbelkörper 18, 19 gegeneinander abstützt.

Eine besonders innige Verbindung der Stützelemente 2 und 3 mit den benachbarten Wirbelkörpern 18 beziehungsweise 19 ergibt sich dadurch, daß beim Befüllen des Hohlraumes 4 ein Teil des Füllmaterials durch den Verbindungskanal 13 in die Vertiefung 12 gelangt und sich im Verbindungsbereich zwischen Außenfläche 11 und benachbartem Wirbelkörper 18 beziehungsweise 19 ausbreitet, nach dem Aushärten unterstützt diese dünne Schicht des eingedrungenen Materials die Verbindung zwischen Stützelement und Wirbelkörper.

Bei der in den Figuren 1 bis 3 dargestellten Ausführungsform werden somit die Wirbelkörper 18 und 19 über das Implantat 1 zu einem einzigen im wesentlichen starren Körper verbunden, die Funktion der entfallenden Bandscheiben wird durch diese Ausführungsform eines Implantates nicht ersetzt.

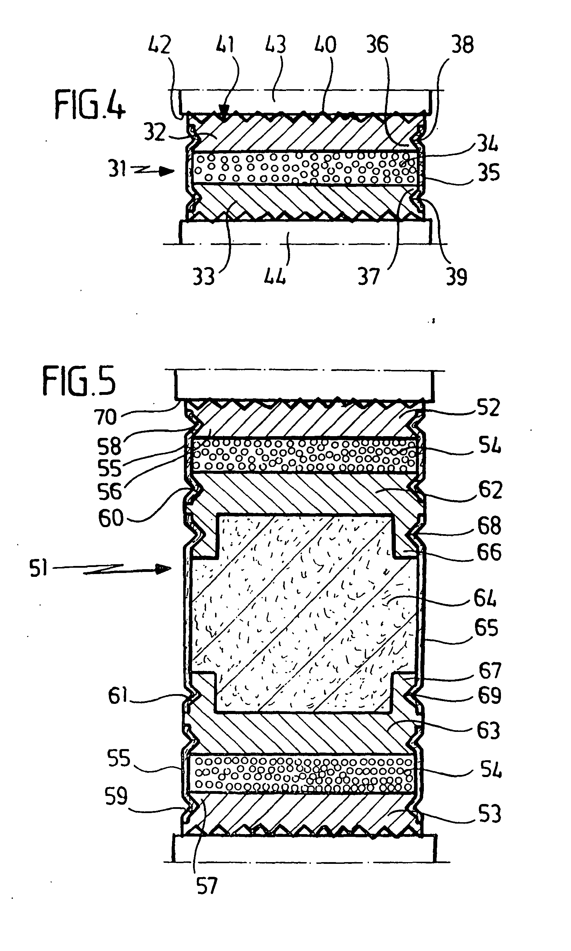

In Figur 4 ist ein weiteres bevorzugten Ausführungsbeispiel eines Implantates 31 dargestellt, welches ebenso wie das Implantat 1 zwei auf gegenüberliegenden Seiten des Implantates angeordnete Stützelemente 32 und 33 aufweist, die zwischen sich einen Hohlraum 34 einschließen. Dieser Hohlraum 34 wird mantelseitig umgeben von einer Außenwand 35, die genau gleich aufgebaut ist wie die Außenwand 5 des Implantates 1, allerdings ist die Erstreckung in Längsrichtung des Implantates 31 deutlich geringer als beim Ausführungsbeispiel der Figuren 1 bis 3.

Auch bei diesem Ausführungsbeispiel weisen die Stützelemente 32 und 33 einander zugewandte, kragenförmige Vorsprünge 36 und 37 mit Umfangsnuten 38 beziehungsweise 39 auf, in die in gleicher Weise die Randbereiche der Außenwand 35 eingelegt und durch nicht dargestellte Bandagen festgelegt sind.

Die Außenfläche 41 der beiden Stützelemente 32 und 33 ist ebenso wie beim Ausführungsbeispiel der Figuren 1 bis 3 mit rippenförmigen Vorsprüngen 40 versehen, es fehlen jedoch eine zentrale Vertiefung und ein Verbindungskanal zum Inneren des Hohlraumes. Bei diesem Implantat ist es auch nicht unbedingt notwendig, Aufnahmeöffnungen für ein Spreizinstrument vorzusehen.

Der Hohlraum 34 wird bei diesem Implantat 31 mit einer fließfähigen Substanz gefüllt, die entweder dauerhaft fließfähig bleibt oder die gummiartig elastisch aushärtet. Eine Füllung mit einem Hydrogel beispielsweise bleibt dauerhaft fließfähig, eine Silikonfüllung dagegen ist eine dauerelastische, nicht fließfähige Füllung. Eine solche Füllung ermöglicht es, die beiden plattenförmigen Stützelemente 32 und 33 gegeneinander in geringem Umfange zu verschwenken, außerdem können die Stützelemente 32 und 33 um die Längsachse des Implantates in geringem Umfange Drehbewegungen ausführen. Das in Figur 4 dargestellte Implantat 31 dient als Ersatz für eine Bandscheibe und wird daher nach Entfernung einer Bandscheibe in den Zwischenwirbelraum 42 zwischen zwei benachbarten Wirbelkörpern 43, 44 eingeschoben. Die Füllung des Hohlraumes kann bei diesem Ausführungsbeispiel des Implantates bereits vor der Implantation oder aber erst nach dem Einschieben in den Zwischenwirbelraum 42 erfolgen, wird der Hohlraum 4 bereits vor dem Einsetzen gefüllt, werden die benachbarten Wirbelkörper 43 und 44 durch geeignete Spreizinstrumente so weit voneinander entfernt, daß das Implantat in den Zwischenwirbelraum eingeschoben werden kann, nach dem Einschieben werden die beiden Wirbelkörper 43 und 44 dann auf die Außenflächen 41 abgesenkt, so daß sie in einem Abstand voneinander gehalten werden, der dem Abstand entspricht, der durch die natürliche, entfernte Bandscheibe erzielt worden ist.

Bei dem Ausführungsbeispiel der Figur 5 sind die Konstruktionen des als Wirbelkörperersatzimplantates dienenden Implantats der Figuren 1 bis 3 und des als Bandscheibenersatz dienenden Implantats der Figur 4 kombiniert.

Das Implantat 51 der Figur 5 weist an gegenüberliegenden Enden zwei Stützelemente 52 und 53 auf, die genauso aufgebaut sind wie die Stützelemente 32 und 33 des Implantates der Figur 4. Auf den einander zugewandten Seiten dieser beiden Stützelemente 52 und 53 schließt sich jeweils ein Hohlraum 54 an, der in gleicher Weise wie der Hohlraum 34 des Implantates 31 von einer Außenwand 55 umgeben ist, die ein balgförmiges, textiles Schlauchstück ist. Jede der beiden Außenwände 55 ist in eine Umfangsnut 58 beziehungsweise 59 an einem kragenförmigen Vorsprung 56 beziehungsweise 57 des Stützelementes 52 und 53 in der beschriebenen Weise festgelegt. Die gegenüberliegenden Enden der beiden Außenwände 55 sind dabei jeweils in eine Umfangsnut 60 beziehungsweise 61 von Zwischenwänden 62 beziehungsweise 63 eingelegt und dort mit nicht dargestellten Bandagen befestigt; diese Zwischenwände 62 und 63 verlaufen parallel zu den plattenförmigen Stützelementen 52 und 53 und schließen die Hohlräume 54 ab.

Die beiden Zwischenwände 62 und 63 bilden zwischen sich ihrerseits einen weiteren Hohlraum 64 aus, der durch eine weitere Außenwand 65 in Form eines textilen, balgförmigen Schlauchstückes abgeschlossen ist. Dieses Schlauchstück ist mit seinen Endabschnitten jeweils in eine weitere Umfangsnut 68 und 69 eines kragenförmigen Vorsprungs 66 beziehungsweise 67 der Zwischenwände 62, 63 eingelegt und dort mittels einer nicht dargestellten Bandage festgelegt.

Auf diese Weise weist dieses Implantat 51 in Längsrichtung des Implantates hintereinander liegend drei Hohlräume 54, 64, 54 auf, wobei der mittlere Hohlraum 64 in Längsrichtung eine wesentliche größere Erstreckung aufweist, als die außenliegenden Hohlräume 54.

Diese beiden außenliegenden Hohlräume 54 werden mit einer dauerhaft fließfähigen oder mit einer elastische Substanz gefüllt und bilden somit Bandscheibenersatzkörper aus, während der mittlere Hohlraum 64 mit einer aushärtbaren Substanz gefüllt wird und somit einen Wirbelersatzkörper ausbildet. Das gesamte Implantat übernimmt somit nach dem Einsetzen in einen Zwischenraum 70 die Funktion der aus diesem Zwischenraum 70 entfernten Teile, nämlich eines Wirbelkörpers und der beiden angrenzenden Bandscheiben.

In dem dargestellten Ausführungsbeispiel sind die Außenwände 55 und 65 durch getrennte Schlauchstücke ausgebildet. Es wäre auch möglich, alle Außenwände durch ein durchgehendes textiles Schlauchstück auszubilden; dadurch würde die Herstellung dieses Implantats noch weiter vereinfacht.

## Patentansprüche

1. Implantat (1) zum Einsetzen in einen Zwischenraum zwischen zwei Wirbelkörpern (18, 19; 43, 44) der Wirbelsäule mit zwei an den zu beiden Seiten des Zwischenraumes angeordneten Wirbelkörpern (18, 19; 43, 44) anlegbaren Stützelementen (2, 3; 32, 33; 52, 53) und mit einem zwischen diesen angeordneten, sich in Längsrichtung des Implantates (1) erstreckenden rohrförmigen Hohlraum (4; 34; 54; 64), der durch eine balgförmige, in Längsrichtung des Implantates (1) verlängerbare und in Umfangsrichtung im wesentlichen nicht dehnbare Außenwand (5; 35; 55; 65) begrenzt wird, **dadurch gekennzeichnet, daß** die Außenwand (5; 35; 55, 65) von einem Schlauchstück aus einem dicht gewebten oder gewirkten textilen Material gebildet wird.

2. Implantat nach Anspruch 1, **dadurch gekennzeichnet, daß** das Schlauchstück aus Fäden aufgebaut ist, die aus Polyethylenterephtalat bestehen.

3. Implantat nach einem der Ansprüche 1 oder 2, **dadurch gekennzeichnet, daß** das Schlauchstück an seinen Enden flüssigkeitsdicht mit den Stützelementen (2, 3; 32, 33; 52, 53) verbunden ist.

4. Implantat nach einem der voranstehenden Ansprüche, **dadurch gekennzeichnet, daß** die Stützelemente (2, 3; 32, 33; 52, 53) plattenförmig ausgebildet sind und sich im wesentlichen quer zur Längsrichtung des Implantates (1; 31; 51) erstrecken.

5. Implantat nach Anspruch 4, **dadurch gekennzeichnet, daß** die Stützelemente (2, 3; 32, 33; 52, 53) an ihrer jeweiligen Außenfläche (11; 41), die einem Wirbelkörper (18, 19; 43, 44) benachbart ist, Vorsprünge (10; 40) trägt, die bei Anlage an dem Wirbelkörper in dessen Knochenmaterial eindringen.

6. Implantat nach einem der Ansprüche 4 oder 5, **dadurch gekennzeichnet**, die Stützelemente (2, 3) Durchbrechungen (13) aufweisen, durch die der Hohlraum (4) mit der Außenfläche (11) der Stützelemente (2, 3) verbunden ist.

7. Implantat nach einem der voranstehenden Ansprüche, **dadurch gekennzeichnet, daß** der Hohlraum (4) einen Zufluß (20) für ein fließfähiges Füllmedium aufweist.

8. Implantat nach einem der voranstehenden Ansprüche, **dadurch gekennzeichnet, daß** der Hohlraum (34; 54) mit einer dauerhaft fließfähigen oder einer elastischen Substanz gefüllt ist.

9. Implantat nach Anspruch 8, **dadurch gekennzeichnet, daß** die dauerhaft fließfähige Substanz ein Hydrogel ist.

10. Implantat nach Anspruch 8, **dadurch gekennzeichnet, daß** die elastische Substanz Silikon ist.

11. Implantat nach einem der Ansprüche 1 bis 7, **dadurch gekennzeichnet, daß** der Hohlraum (4; 64) mit einer fließfähigen, aushärtbaren Substanz gefüllt ist.

12. Implantat nach Anspruch 11, **dadurch gekennzeichnet, daß** die fließfähige, aushärtbare Substanz Knochenzement ist.

13. Implantat nach einem der voranstehenden Ansprüche, **dadurch gekennzeichnet, daß** sich in Längsrichtung des Implantates (51) zwischen den Stützelementen (52, 53) mindestens zwei Hohlräume (54, 64) befinden, die von einer schlauchförmigen Außenwand (55, 65) aus einem dicht gewebten oder gewirkten textilen Material begrenzt werden.

14. Implantat nach Anspruch 13, **dadurch gekennzeichnet, daß** bei einem Implantat mit zwei Hohlräumen ein Hohlraum mit einer dauerhaft fließfähigen oder einer elastischen Substanz gefüllt ist und der andere mit einer fließfähigen, aushärtbaren Substanz.

15. Implantat nach Anspruch 13, **dadurch gekennzeichnet, daß** bei einem Implantat (51) mit drei in Längsrichtung aneinander anschließenden Hohlräumen (54, 64, 54) der mittlere Hohlraum (64) mit einer fließfähigen, aushärtbaren Substanz gefüllt ist und die beiden äußeren Hohlräume (54) mit einer dauerhaft fließfähigen oder elastischen Substanz.

16. Implantat nach einem der Ansprüche 14 oder 15, **dadurch gekennzeichnet, daß** die Ausdehnung des mit einer aushärtbaren Substanz gefüllten Hohlraumes (64) in Längsrichtung des Implantates (51) größer ist als die des mit einer dauerhaft fließfähigen oder elastischen Substanz gefüllten Hohlraumes (54).

## Claims

1. An implant (1) for insertion into a space between two vertebrae (18, 19; 43, 44) of the spinal column, with two supporting members (2, 3; 32, 33; 52, 53) which are placeable against the vertebrae (18, 19; 43, 44) arranged on either side of the space and with a tubular cavity (4; 34; 54; 64) which is arranged between the supporting members (2, 3; 32, 33; 52, 53) and extends in the longitudinal direction of the implant (1) and is bounded by a bellows-type outer wall (5; 35; 55; 65) which is extensible in the longitudinal direction of the implant (1) and substantially non-expandable in the circumferential direction, **characterised in that** the outer wall (5; 35; 55; 65) is formed by a tubular part made of a tightly woven or knitted textile material.

2. An implant according to claim 1, **characterised in that** the tubular part is constructed from threads made of polyethylene terephthalate.

3. An implant according to either one of claims 1 and 2, **characterised in that** the ends of the tubular part are connected to the supporting members (2, 3; 32, 33; 52, 53) in a liquid-tight manner.

4. An implant according to any one of the preceding claims, **characterised in that** the supporting members (2, 3; 32, 33; 52, 53) are plate-shaped and extend substantially transversely to the longitudinal direction of the implant (1; 31; 51).

5. An implant according to claim 4, **characterised in that** the supporting members (2, 3; 32, 33; 52, 53) carry projections (10; 40) on their respective outer surface (11; 41) adjacent to a vertebra (18, 19; 43, 44), which projections (10; 40) penetrate the bone material of the vertebra when placed thereagainst.

6. An implant according to either one of claims 4 and 5, **characterised in that** the supporting members (2, 3) have openings (13), by means of which the cavity (4) is connected to the outer surface (11) of the supporting members (2, 3).

7. An implant according to any one of the preceding claims, **characterised in that** the cavity (4) has an inlet (20) for a fluid filling medium.

8. An implant according to any one of the preceding claims, **characterised in that** the cavity (34; 54) is filled with a permanently fluid or a resilient substance.

9. An implant according to claim 8, **characterised in that** the permanently fluid substance is a hydrogel.

10. An implant according to claim 8, **characterised in that** the resilient substance is silicone.

11. An implant according to any one of claims 1 to 7, **characterised in that** the cavity (4; 64) is filled with a fluid, hardenable substance.

12. An implant according to claim 11, **characterised in that** the fluid, hardenable substance is bone cement.

13. An implant according to any one of the preceding claims, **characterised in that** at least two cavities (54, 64) are arranged in the longitudinal direction of the implant (51) between the supporting members (52, 53) and are bounded by a tubular outer wall (55, 56) made of a tightly woven or knitted textile material.

14. An implant according to claim 13, **characterised in that**, in the case of an implant with two cavities, one cavity is filled with a permanently fluid or a resilient substance and the other cavity is filled with a fluid, hardenable substance.

15. An implant according to claim 13, **characterised in that**, in the case of an implant (51) with three cavities (54, 64, 54) following one another in the longitudinal direction, the middle cavity (64) is filled with a fluid, hardenable substance and the two outer cavities (54) are filled with a permanently fluid or resilient substance.

16. An implant according to either one of claims 14 and 15, **characterised in that** the extension, in the longitudinal direction of the implant (51), of the cavity (64) filled with a hardenable substance is greater than that of the cavity (54) filled with a permanently fluid or resilient substance.

## Revendications

1. Implant (1) à mettre en place dans un intervalle entre deux corps vertébraux (18, 19 ; 43, 44) de la colonne vertébrale comprenant des éléments de soutien (2, 3 ; 32, 33 ; 52, 53) susceptibles d'être appliqués contre les corps vertébraux (18, 19 ; 43, 44) agencés des deux côtés de l'intervalle, et comprenant une cavité (4 ; 34 ; 54 ; 64) de forme tubulaire agencée entre ces éléments de soutien et s'étendant en direction longitudinale de l'implant (1), ladite cavité étant délimitée par une paroi extérieure (5 ; 35 ; 55 ; 65) en forme de soufflet, capable de s'allonger en direction longitudinale de l'implant (1), mais pas sensiblement extensible en direction périphérique, **caractérisé en ce que** la paroi extérieure (5 ; 35 ; 55 ; 65) est formée par un morceau de tuyau en un matériau textile à tissage serré ou tricoté.

2. Implant selon la revendication 1, **caractérisé en ce que** le morceau de tuyau est réalisé à partir de fibres en polyéthylène téréphtalate.

3. Implant selon l'une des revendications 1 au 2, **caractérisé en ce que** le morceau de tuyau est relié à ses extrémités avec les éléments de soutien (2, 3 ; 32, 33 ; 52, 53) de façon étanche aux liquides.

4. Implant selon l'une des revendications précédentes, **caractérisé en ce que** les éléments de soutien (2, 3 ; 32, 33 ; 52, 53) sont réalisés sous forme de plaquettes et s'étendent essentiellement transversalement à la direction longitudinale de l'implant (1 ; 31 ; 51).

5. Implant selon la revendication 4, **caractérisé en ce que** les éléments de soutien (2, 3 ; 32, 33 ; 52, 53) portent, sur leur surface extérieure respective (11 ; 41), voisine d'un corps vertébral (18, 19 ; 43, 44), des saillies (10 ; 40) qui, lors de la venue en contact contre le corps vertébral, s'enfoncent dans son matériau osseux.

6. Implant selon l'une des revendications 4 ou 5, **caractérisé en ce que** les éléments de soutien (2, 3) comportent des traversées (13) via lesquelles la cavité (4) est reliée à la surface extérieure (11) des éléments de soutien (2, 3).

7. Implant selon l'une des revendications précédentes, **caractérisé en ce que** la cavité (4) comporte une amenée (20) pour un fluide de remplissage capable de s'écouler.

8. Implant selon l'une des revendications précédentes, **caractérisé en ce que** la cavité (34 ; 54) est remplie d'une substance capable durablement de s'écouler ou d'une substance élastique.

9. Implant selon la revendication 8, **caractérisé en ce que** la substance capable durablement de s'écouler est un hydro-gel.

10. Implant selon la revendication 8, **caractérisé en ce que** la substance élastique est du silicone.

11. Implant selon l'une des revendications 1 à 7, **caractérisé en ce que** la cavité (4 ; 64) est remplie d'une substance capable de s'écouler et durcissable.

12. Implant selon la revendication 11, **caractérisé en ce que** la substance capable de s'écouler et durcissable est du ciment osseux.

13. Implant selon l'une des revendications précédentes, **caractérisé en ce que**, en direction longitudinale de l'implant (51) et entre les éléments de soutien (52, 53), se trouvent au moins deux cavités (54, 64) qui sont délimitées par une paroi extérieure (55, 65) en forme de tuyau en un matériau textile à tissage serré ou tricoté.

14. Implant selon la revendication 13, **caractérisé en ce que**, dans un implant avec deux cavités, une cavité est remplie d'une substance capable durablement de s'écouler ou d'une substance élastique, et l'autre cavité est remplie d'une substance capable de s'écouler et durcissable.

15. Implant selon la revendication 13, **caractérisé en ce que**, dans un implant avec trois cavités (54, 64, 54) qui se suivent mutuellement en direction longitudinale, la cavité médiane (64) est remplie d'une substance capable de s'écouler et durcissable, et les deux cavités extérieures (54) sont remplies d'une substance capable durablement de s'écouler ou d'une substance élastique.

16. Implant selon l'une des revendications 14 ou 15, **caractérisé en ce que** l'extension de la cavité (64) remplie avec d'une substance durcissable en direction longitudinale de l'implant (51) est supérieure à celle de la cavité (54) remplie d'une substance capable durablement de s'écouler ou d'une substance élastique.
